# FASCICULE DE BREVET EUROPEEN

(11) **EP 0 502 172 B1**
(45) Date de publication et mention de la délivrance du brevet: **20.08.1997**
(21) Numéro de dépôt: 91917771.7
(22) Date de dépôt: 02.10.1991
(51) Int. Cl.: C12N 5/08, A61L 15/32, A61L 27/00

(54) **BIOMATERIAU A BASE DE COLLAGENE ET SES APPLICATIONS**
BIOMATERIAL AUF KOLLAGENBASIS UND SEINE ANWENDUNGEN
BIOMATERIAL BASED ON COLLAGEN AND ITS APPLICATIONS

(30) Priorité: 02.10.1990 FR 9012135
(43) Date de publication de la demande: 09.09.1992
(73) Titulaire: IMEDEX S.A., F-69630 Chaponost (FR); PASTEUR MERIEUX SERUMS ET VACCINS, 69007 Lyon (FR)
(72) Inventeur: TINOIS, Estelle, F-69160 Tassin-La-Demi-Lune (FR); TIOLLIER, Jérôme, F-69009 Lyon (FR); DUMAS, Henri, F-69005 Lyon (FR); TARDY, Michel, F-69005 Lyon (FR)
(74) Mandataire: Bernasconi, Jean
(86) Numéro de dépôt international: FR9100773
(87) Numéro de publication internationale: WO9206179

(56) Documents cités:
- WO-A-89/03392
- WO-A-89/08467
- The Journal of Investigative Dermatology, volume 95, no. 3, septembre 1990, (Baltimore, PA, US) M.S. Wilke et al.: "Human keratinocytes adhere to a unique heparin-binding peptide sequence within the triple helical region of type IV collagen", voir pages 264-270
- Biological Abstracts, volume 84, no. 9, 1987, (Philadelphia, PA, US) E.M. Tinois et al.: "Growth and differentiation of human keratinocytes on extracellular matrix", & Arch. Dermatol. Res., 279(4): 241-246, 1987, see p. AB-564, abstract no. 88152
- Experimental Cell Research, volume 193, 1991, Academic Press (Duluth, Minn., US) E. Tinois et al.: "In vitro and post-transplantation differentiation of human keratinocytes grown on the human type IV collagen film of a bilayered dermal substitute", voir pages 310-319

## Description

La présente invention concerne un biomatériau à base de collagène et des applications de celui-ci.

Au cours des vingt dernières années, des progrès majeurs ont été réalisés dans le domaine de la culture de cellules épidermiques. Ces progrès ont permis la culture d'un nombre croissant de cellules (références bibliographiques 1 et 2) et ont fourni des indications importantes sur les facteurs régulant la croissance et la différenciation terminale de l'épiderme. Différents systèmes ont été développés qui permettent de moduler à la fois la croissance et la différenciation (2, 3, 4). Afin d'étudier davantage la régulation de ces phénomènes biologiques dans le dessein d'étudier les interactions derme-épiderme et de reconstituer un tissu analogue à la peau, on a cultivé des cellules sur des substituts dermiques plus ou moins complexe (références 5 à 13). Du fait de la faible capacité de fixation des kératinocytes sur le substitut dermique, la formation de la membrane basale in vitro a fait l'objet de nombreux travaux. Les seuls résultats publiés au sujet de la reconstitution d'une membrane basale presque complète par des kératinocytes humains dispersés en culture sur du collagène ont fait état de cultures de longue durée (40 à 60 jours) (14, 15). Des auteurs ont rapporté le rôle du collagène de type IV dans la culture des kératinocytes (22, 23, 24). Document (25) montre l'effet favorable de l'utilisation de matrices extracellulaires (ECM) dans la liaison, le développement et la différenciation de kératinocytes humaines in vitro.

Par ailleurs, on a déjà réalisé (EP-A-0.357.755) un patch de chirurgie viscérale formé de deux couches de collagène superposées et associées intimement, à savoir une couche poreuse de collagène fibreux et un film de collagène, le collagène pouvant notamment être de type III + I ou de type IV.

La présente invention a pour objectif de fournir un biomatériau comportant des cellules épidermiques différenciées et notamment un biomatériau de ce type dont la structure se rapproche sensiblement de celle de la peau.

Un autre objectif de l'invention est de fournir un biomatériau utile en pharmacologie, toxicologie et cosmétologie et/ou pour la protection de plaies, brûlures ou la cicatrisation.

La déposante a trouvé de façon surprenante qu'une matrice de collagène de type IV permettait t la culture de cellules épidermiques, notamment de kératinocytes, en jouant le rôle d'un support ou substitut dermique et d'obtenir très rapidement un biomatériau dont la structure générale est proche de celle de la peau avec, notamment, formation de ce qu'on peut assimiler à une membrane basale et de cellules cornées.

L'invention a donc pour objet un biomatériau à base de collagène, comportant, intimement associés, un support ou substitut dermique à base de collagène de type IV et un épithélium épidermique à cellules différenciées obtenu par culture de cellules épidermiques sur ledit support ou substitut.

De façon avantageuse, le support ou substitut dermique peut comporter en outre une sous-couche de collagène de type I + III sous la forme d'un treillis poreux.

Les collagènes mis en oeuvre peuvent être réticulés ou natifs ou peuvent être un mélange approprié de collagène natif et de collagène réticulé.

Le collagène de type IV peut être notamment d'origine placentaire et préparé selon le brevet EP-A-0.214.035.

De préférence, les cellules épidermiques de colonisation sont des kératinocytes, notamment des kératinocytes humains normaux d'adulte (KHNA).

L'épithélium de ce biomatériau présente notamment les caractéristiques suivantes :
a) à partir de 6 jours de culture, une bonne reconstitution des structures d'ancrage au niveau de la jonction dermo-épidermique : nombreux hémidesmosomes bien individualisés (plaque d'ancrage avec tonofilaments s'y insérant, plaque dense sous-basale, filaments d'ancrage) et une déposition en bande, régulière, de matériel extracellulaire entre la surface du collagène de type IV et la membrane plasmique ressemblant à une lamina densa.
b) 3 compartiments cellulaires :
   - une assise de cellules basales,
   - plusieurs assises de cellules intermédiaires reliées entre elles par des desmosomes, contenant des grains de kératohyaline pour les plus superficielles d'entre elles,
   - un stratum corneum.

L'invention a également pour objet l'application de ce biomatériau à des tests pharmacologique, toxicologique ou cosmétologique, notamment sous forme d'un kit d'évaluation in vitro.

L'invention a encore pour objet l'application de ce biomatériau à la protection/cicatrisation de plaies et brûlures.

L'invention va être maintenant décrite plus en détail à l'aide d'un procédé de préparation d'un biomatériau selon l'invention et d'essais in vitro et in vivo.

### Préparation des collagènes.

La préparation du collagène IV, natif ou oxydé, est réalisée suivant le procédé décrit dans le brevet EP-A-0.214.035.

Les collagènes de type I et III peuvent être préparés selon tout procédé connu.

### Préparation du substitut dermique.

Le substitut dermique comporte des collagènes de type I + III oxydés et lyophilisés, recouverts par une couche d'un mélange de collagène de type IV et de collagène de type IV oxydé. Pour la préparation du substitut dermique, on a ajouté une solution d'acide periodique (concentration finale 0,002 M) à une solution à 10 mg/ml de collagènes de type I et de type III dans de l'acide hydrochlorique 0,01 N. Les collagènes oxydés ont été récupérés par précipitation au phosphate (Na₂HPO₄). Le précipité a été lavé plusieurs fois avec un tampon phosphate (Na₂HPO₄) et récupéré par filtration sur une toile de Nylon. Le produit obtenu a été mis sous forme de feuilles, lyophilisé et comprimé. L'oxydation induite par l'acide periodique conduit à la formation d'une matrice de collagène réticulé (brevet US-4.931.546).

On a réalisé une solution de collagène oxydé de type IV (IVox) en ajoutant de l'acide periodique (concentration finale 0,02 M) à une solution à 20 mg/ml de collagène de type IV dans de l'acide hydrochlorique 0,01 N. Après 2 heures d'incubation, le collagène de type IVox a été dialysé contre de l'acide hydrochlorique 0,01 N.

Un mélange formé de 10 mg/ml d'une préparation de collagène de type IV et de 10 mg/ml d'une préparation de collagène de type IV oxydé (4:1) (IV/IVox) a été déposé à la surface de l'éponge de collagène de type I + III, puis a été séché. La stérilisation du substitut dermique a été réalisée par irradiation gamma (25 kgray).

### Culture de KHNA (kératinocytes humains normaux d'adulte).

On a préparé des suspensions de cellules épidermiques humaines par des procédés standards de trypsination à partir d'échantillons de peau d'adultes normaux prélevés lors d'opérations de chirurgie plastique. Les cellules ont été propagées sur des couches de cellules nourricières 3T3 gamma-irradiées, selon le procédé de H. Green (1).

Le milieu de culture était le DMEM (Gibco Laboratories, Grand Island, NY, USA) et HAM F12 (Gibco) (3:1) supplémenté avec 10 % de sérum de veau foetal (Boehringer Mannheim, Meylan, France), 1,8.10⁻⁴ M d'adénine, 5 µg/ml d'insuline, 2.10⁻⁹ M de triiodothyronine, 5 µg/ml de transferrine, 0,4 µg/ml d'hydrocortisone, 10⁻¹⁰ M de toxine cholérique et 10 ng/ml de facteur de croissance épidermique, tous obtenus de SIGMA, USA (milieu complet). A confluence, les kératynocytes ont été trypsinés et stockés dans de l'azote liquide. Des échantillons circulaires de substitut dermique, de 1,6 cm de diamètre, ont été placés dans des plaques de culture à 24 puits (Falcon) et ont été comprimés pour adhérer à la matière plastique. Des kératinocytes en suspension ont été ensemencés directement sur ces échantillons et mis en contact d'un milieu de culture (on a ensemencé 5.10⁴ cellules par cm² de façon à obtenir la confluence en 4 jours). Les cultures ont été incubées à 37°C en atmosphère à 5 % de CO₂ et le milieu de culture a été changé tous les deux jours. Lorsque la confluence des cellules épidermiques a été atteinte, on a retiré le substitut de peau obtenu pour un examen sous microscope ou pour transplantation chez la souris. Des substituts dermiques ont été placés, 5 jours après mise sur plaque, sur des grilles en acier de façon à amener la culture de kératynocytes à l'interface air-liquide. Les cellules sont ensuite exposées à l'air et alimentées à travers le substitut dermique.

La partie supérieure du substitut dermique est une couche de collagène de type IV-IVox. Pour étudier la capacité du biomatériau à permettre la croissance de cellules épidermiques, on a cultivé des KHNA sur une couche transparente analogue de collagène IV-IVox réalisée sur des plaques à 6 puits, de façon à permettre une observation microscopique directe. Les cellules ont été ensemencées en suspension sur les couches dans un milieu complet ou dans un milieu sans sérum. Le milieu sans sérum était du DMEM et du HAM F12 (1:1) (Gibco) supplémenté avec 0,5 µg/ml d'hydrocortisone, 5 µg/ml d'insuline, 3.10⁻⁸ M de sélénite (SIGMA), 5 µg/ml de transferrine, 1 mg/ml d'albumine (Institut Mérieux, Lyon, France), 5 µg/ml de fibronectine (Institut Mérieux). De fortes densités d'ensemencement, 10⁵, 5.10⁴, 3.10⁴ cellules/cm², ont été testées dans un milieu complet et une faible densité d'ensemencement, 8.10³ cellules/cm², dans du milieu sans sérum.

### Différenciation des cellules épidermiques.

Pour l'histologie, les échantillons ont été fixés dans un milieu de fixation de Bouin, déshydratés dans l'alcool et enrobés de paraffine et de méthacrylate. Des coupes ont été colorées avec de l'HES (hématoxyline-éosine-safran). Des études d'immunohistochimie ont été réalisées sur des coupes déparaffinées ou congelées, en utilisant une technique à l'avidine-biotine-phosphatase alcaline ou par immunofluorescence indirecte.

La différenciation épidermique a été étudiée en utilisant 3 anticorps monoclonaux : anticorps anti-kératine KL1 (Immunotech, Marseille, France) ; AKH1, un anticorps monoclonal contre la profilaggrine/filaggrine humaine (Biomedical Tech. Inc., Soughton, USA), et un anticorps polyclonal de lapin contre l'involucrine (Biomedical Tech.). On a utilisé un anticorps monoclonal de souris contre la microglobuline béta-2 (Immunotech) comme marqueur d'antigène humain de classe I (16 et 17).

Pour la microscopie électronique par transmission, des échantillons ont été fixés dans 1 % de glutaraldéhyde, 0,5 % de paraformaldéhyde, 0,1 M de tampon phosphate, préfixé dans 1 % de tétroxyde d'osmium, déshydraté dans de l'alcool et enrobé dans un milieu époxy. Des coupes ultrafines sont observées et photographiées avec un microscope électronique à transmission JEOL 1200 EX (18).

### Transplantation à une souris athymique.

La technique de greffage est issue de celle décrite précédemment dans la référence bibliographique 19. En bref, 17 souris athymiques congénitalement (Swiss nu/nu, Iffa Credo, Lyon, France) (âgées de 6 à 10 semaines) ont été anesthésiées à l'aide de pentobarbital de sodium, et un siège de greffe circulaire, de 1,5 cm de diamètre, a été préparé sur la face dorso-latérale de chaque animal en retirant délicatement l'épiderme et le derme, tout en laissant le tissu de vascularisation sur la fascia.

Le greffon, dont les bords sont placés sous la peau adjacente de la souris, est recouvert d'une gaze imprégnée de vaseline et d'une capsule en matière plastique (pour 4 souris sur 17) maintenue en place par 4 sutures croisées et protégée par un bandage. Pour 7 autres souris, les capsules en matière plastique sont omises et les greffons sont maintenus en place par un bandage compressif. En variante, pour les 6 souris restantes, les capsules en matière plastique sont remplacées par une chambre de transplantation en silicone (Renner GmbH, Darmstadt, RFA) qui a été placée sur le greffon et qui est maintenue en place par des clips de 9 mm. Après 14, 20 ou 30 jours, le bandage, la capsule et la gaze ont été retirés et les greffons ont été excisés chirurgicalement et préparés pour la microscopie optique ou électronique.

### Etude de la membrane basale.

La formation de la membrane basale a été étudiée par immunofluorescence indirecte sur des coupes congelées et par microscopie électronique à transmission.

On a utilisé l'anticorps à la laminine (Institut Pasteur), l'anticorps au collagène de type IV (Institut Pasteur) ou l'anticorps monoclonal de souris au collagène humain de type IV (HEYL) et le sérum de patients atteints de Bullous Pemphigoid (BP) pour détecter les trois composants de la jonction derme-épiderme.

### RESULTATS

### Le substitut dermique.

Le substitut dermique est formé d'une couche poreuse (diamètre des pores : 50 à 100 microns) et fibreuse de collagène de type I et III, de 400 microns d'épaisseur, recouverte d'une couche dense de collagène de type IV/IVox de 10 à 20 microns d'épaisseur.

Lorsqu'il est recouvert d'épithélium multicouches, le substitut peut être facilement manipulé sans support.

Par immunofluorescence indirecte, l'anticorps au collagène de type IV met en évidence une fine couche à la surface de la couche de type I + III et de fins filaments inclus dans la couche de type I + III montrant que le collagène de type IV/IVox a légèrement pénétré dans la couche de type I + III.

### Croissance et différenciation in vitro des KHNA.

Les KHNA se fixent rapidement au collagène, s'étendent et se divisent pour former un épithélium confluent. La confluence est obtenue en 3, 4 et 5 jours lorsque l'on ensemence respectivement 10⁵, 5.10⁴ et 3.10⁴ cellules/cm². Dans un milieu de culture sans sérum, une densité d'ensemencement de 8.10³ cellules/cm² permet une confluence en 13 jours.

Par des méthodes histologiques et par microscopie électronique, on voit que la différenciation des cellules épidermiques se produit entre le 6ème et le 25ème jours après mise en culture. L'examen histologique après 14 jours de culture montre une feuille épithéliale multicouches formée d'une couche de cellules basales bien organisée et de plusieurs couches de cellules suprabasales comportant plus de cellules aplaties et anucléées. Des grains de kératohyaline clairsemés sont observés dans le cytoplasme des cellules en dessous des couches de cellules anucléées. Lorsque les cultures sont exposées à l'air, on observe une couche cornée épaisse. On observe occasionnellement une parakératose.

Au jour 6, une couche de cellules basales composée de petites cellules cubiques comportant un noyau distinct, des organites cytoplasmiques, des filaments intermédiaires et des tonofilaments, peut être vue au microscope électronique. Cette couche est recouverte de couches suprabasales formées de cellules plus allongées contenant de nombreux organites et filaments intermédiaires mais pas de grains de kératohyaline. La stratification augmente ensuite avec l'âge de la culture. Les couches de cellules sont étroitement associés avec des desmosomes bien structurés et des espaces inter-cellulaires étroits. Les grains de kératohyaline sont clairsemés, ont une forme arrondie et s'observent dans les couches de cellules intermédiaires supérieures. Ensuite, la stratification n'augmente pas de façon significative mais on peut observer des cellules anucléées et un peu desquamantes.

### Différenciation in vivo des kératinocytes.

On observe un épiderme bien stratifé seulement dans le cas où les greffons ont été maintenus dans des chambres de greffons en matière plastique ou en silicone inhibant la migration des cellules épidermiques murines. Au jour 14, la couche de collagène I + III du substitut dermique commence à être infiltrée par des cellules inflammatoires et des fibroblastes. Au jour 30, les fibres de collagène de type I + III ne se présentent que par endroits, colonisées par de nombreux fibroblastes et quelques cellules mononucléaires. La dégradation de la couche de collagène de type I + III conduit de façon hétérogène à des fibres de collagène presque intactes dans certaines régions ou à un tissu réorganisé contenant entre autres des fibres de collagène humain éparpillées. La couche de collagène de type IV/IVox reste sensiblement intacte et non infiltrée. Le collagène de type IV/IVox semble donc résistant à la dégradation dans ces conditions occlusives. Lorsque le substitut dermique est greffé dans des conditions non occlusives, sa dégradation est plus rapide. Après la greffe, l'épithélium développe à la fois une couche granulaire et une couche cornée dans les deux semaines. L'épiderme greffé n'est pas hypertrophique comme cela est communément décrit dans le cas d'épithélium de culture ou de substitut de peau greffés (20 et 21).

Deux semaines après la greffe, on observe de nombreux grains de kératohyaline dans la couche viable supérieure et un stratum corneum avec une faible orthokératose. Au jour 20, la microglobuline béta-2 est exprimée dans la couche de cellules basales et, de façon faible, dans quelques couches suprabasales. Au jour 30, les couches de cellules basales et de cellules suprabasales sont fortement marquées.

Au jour 14, la coloration obtenue avec l'anticorps KL1 a été observée pour toutes les couches épidermiques. La couche de cellules basales commence à être partiellement négative au jour 20. Le marquage profilaggrine/filaggrine est limité à la couche granulaire, comme on l'observe pour la peau humaine normale. L'expression d'involucrine se produit en position suprabasale aux jours 14, 20 et 30. Néanmoins, aux jours 30 et 55, cette expression est largement limitée aux couches intermédiaires et supérieures de Malpighi, et aux couches granulaires au jour 30.

### Etude de la jonction derme-épiderme.

### In vitro :

La microscopie électroniques montre de nombreuses structures du type hémidesmosome à la jonction entre la couche de collagène de type IV/IVox et les cellules basales ainsi qu'au jour 6 après mise en culture. La jonction derme-épiderme est linéaire, sans crêtes. On observe une plaque intracellulaire opaque aux électrons le long de la membrane de cellules basales dans laquelle s'insèrent des filaments intermédiaires et une couche dense subbasale. On peut distinguer des filaments d'ancrage. La surface du film apparaît comme une ligne dense et une bande opaque aux électrons ressemblant à une lamina densa semble la recouvrir. Cela ressemble à une jonction épiderme-derme dans la peau d'un foetus humain à un stade intermédiaire du développement.

Par immunofluorescence, l'antigène BP est exprimé à partie du 6ème jour de culture tandis que la laminine qui n'était pas détectable à ce stade peut l'être à partir du 12ème jour. Les deux marqueurs antigéniques s'observent sur la face basale des cellules basales sous forme d'une fluorescence discontinue. La réaction d'immunofluorescence utilisant l'anticorps au collagène de type IV montre une fluorescence sur toute l'épaisseur de la couche de collagène de type IV/IVox.

### In vivo :

Aux jours 30 et 55 après la transplantation, la structure fine de la membrane basale est plus nette qu'en culture in vitro puisque le collagène IV/IVox apparaît moins dense. De nombreux hémidesmosomes bien différenciés et une lamina densa sont mis en évidence. Dans certaines régions, la couche de collagène de type IV/IVox semble partiellement dégradée par les cellules épidermiques. Dans ces régions, au 30ème jour, des filaments d'ancrage peuvent apparaître sous la lamina densa. Au 55ème jour, le tissu conjonctif sous la lamina densa est mieux organisé et les filaments d'ancrage sont bien structurés. En microscopie optique, la jonction derme-épiderme apparaît légèrement ondulée et, en microscopie électronique, l'interface derme-épiderme des cellules basales forme une membrane plasmique ondulée dans sa partie basale.

La qualité de l'épiderme obtenu permet d'appliquer le matériau aux tests d'évaluation toxicologiques, pharmacologiques, et cosmétologiques, selon les procédures usuelles pratiquées sur des biopsies de peau humaine et animale.

### REFERENCES BIBLIOGRAPHIQUES.

1. Green H., Kehinde O. & Thomas J., Proc. Natl. Acad. Sc., USA 76 (1979) 5665
2. Boyce S. & Ham RG., J. Invest. Dermatol. 81 (1983) 33s
3. Hennings H., Michael D., Cheng C., Steward S., Holbrook K. & Yuspa SH., Cell 19 (1980) 245
4. Asselineau D., Bernard B., Bailly C. & Darmon M. Exp. Cell. Res. 159 (1985) 536
5. Bell E., Sher S., Hull B., Merill C., Rosen S., Chamson A., Asselineau D., Dubertret L., Coulomb B., Lapiere C., Nusgens B. & Neveux Y., J. Invest. Dermatol. 81 (1983) 2s
6. Coulomb B., Saiag GP., Bell E., Breitburd F., Lebreton C., Heslan M. & Dubertret T., Br. J. Dermatol. 114 (1986) 91
7. Prunieras M., Regnier M. & Woodley D., J. Invest. Dermatol. 81 (1983) 28s
8. Regnier M., Desbas C., Bailly C. & Darmon M., In vitro 24 (1988) 625
9. Regnier M. & Darmon M., In vitro, 25 (1989) 1000
10 Lenoir M-C., Bernard B., Pautrat G., Darmon M. & Shroot B., Dev. Biol. 130 (1988) 610
11. Freeman AE.. Igel HJ., Herman BJ & Kleinfeld KL., In vitro 12 (1976) 352
12. Yannas IV. & Burke JF., J. Biomed. Mater. Res. 14 (1980) 65
13. Boyce S. & Hansbrough J., Surgery 103 (1988) 421
14. Hirone T. & Taniguchi S., Cur. Prob. Dermatol. 10 (1980) 159
15. Chamson A., Germain N., Claudy A., Perier C. & Frey J., Arch. Dermatol. 281 (1989) 267
16. Forsum U. & Tjemlund UM., Acta Venereol (Stockh) 57 (1977) 121
17. Mauduit G., Vincent CL., Gielen V., Faure M., Demiden A. & Thivolet J., Tissue antigens 29 (1987) 65
18. Garrone R. & Tiollier J., dans Bienvenu, Grimaud, Laurent, Walterde, Gruyter (Eds), Marker proteins, vol. 3 1986. pp 357-361
19. Tinois E., Faure M., Kanitakis J., Ramirez-Bosca A., Nguyen C., Tardy M., Tayot JL. & Thivolet J., Epithelia 1(1987) 141
20. Faure M., Mauduit G., Schmitt D., Kanitakis J., Demidem A. & Thivolet J., Br. J. Dermatol. 116 (1987) 161
21. Ramirez-Bosca A., Tinois E., Faure M., Kanitakis J., Roche P. & Thivolet J., J. Invest. Dermatol. 91 (1988) 36
22. Murray JC., Stingl G., Kleinman HK., Martin GR. & Katz SI., J. Cell. Biol. 80 (1979) 197
23. Kleinman HK., Murray JC., Mc Goodwin EB. & Martin GR., J. Invest. Dermatol. 71 (1978) 9
24. Woodley D., Kimberly C. & O'Keefe J., J. Invest. Dermatol. 94 (1990) 139.
25. E. Tinois et al., Arch. Dermatol. Res., 279 (1987), 241-246

## Revendications

1. Biomatériau à base de collagène, caractérisé en ce qu'il comporte, intimement associés, un support ou substitut dermique comprenant une couche à base essentiellement de collagène de type IV et un épithélium épidermique à cellules différenciées obtenu par culture de cellules épidermiques sur ladite couche dudit support ou substitut dermique.

2. Biomatériau selon la revendication 1, caractérisé en ce que le support ou substitut dermique comporte en outre une sous-couche d'au moins un autre type de collagène.

3. Biomatériau selon la revendication 2, caractérisé en ce que ladite sous-couche comporte des collagènes des types I et III.

4. Biomatériau selon l'une quelconque des revendications 1 à 3, caractérisé en ce que les cellules épidermiques sont des kératinocytes.

5. Biomatériau selon l'une quelconque des revendications 1 à 4, caractérisé en ce que les collagènes sont des collagènes natifs ou réticulés ou des mélanges de collagène natif et de collagène réticulé.

6. Application du biomatériau selon l'une quelconque des revendications 1 à 5 dans un test d'évaluation in vitro en toxicologie, pharmacologie ou cosmétologie.

7. Biomatériau selon l'une quelconque des revendications 1 à 5 pour la protection des plaies et brûlures ou formant agent de cicatrisation.

## Patentansprüche

1. Biomaterial auf der Basis von Kollagen, dadurch gekennzeichnet, daß es, in inniger Weise verbunden, einen Träger oder einen Dermisersatz aufweist, der eine Schicht im wesentlichen auf der Basis vom Kollagen Typ IV und einem Epidermisepithel aus differenzierten Zellen umfaßt, wobei das Epidermisepithel erhalten wird durch Kultivieren von Epidermiszellen auf dieser Schicht des Trägers oder Dermisersatzes.

2. Biomaterial nach Anspruch 1, dadurch gekennzeichnet, daß der Träger oder der Dermisersatz ferner eine Unterschicht aus wenigstens einem anderen Kollagen aufweist.

3. Biomaterial nach Anspruch 2, dadurch gekennzeichnet, daß diese Unterschicht Kollagene der Typen I und III aufweist.

4. Biomaterial nach einem der Ansprüche 1 bis 3, dadurch gekennzeichnet, daß die Epidermiszellen Keratinozyten sind.

5. Biomaterial nach einem der Ansprüche 1 bis 4, dadurch gekennzeichnet, daß die Kollagene nativ oder vernetzt oder Mischungen eines nativen Kollagens und vernetzten Kollagens sind.

6. Verwendung des Biomaterials nach einem der Ansprüche 1 bis 5 zur in vitro Bewertung in der Toxikologie, Pharmakologie oder Kosmetik.

7. Biomaterial nach einem der Ansprüche 1 bis 5 für den Schutz von Wunden und Verbrennungen oder zur Herstellung eines Vernarbungsmittels.

## Claims

1. Biomaterial based on collagen, characterized in that it comprisee, intimately linked, a support or skin substitute comprising a layer based easentially on type IV collagen and an epidermal epithelium having differentiated cells which is obtained by culturing epidermal cells on the said layer of the said support or skin substitute.

2. Biomaterial according to Claim 1, characterized in that the support or skin substitute comprises, in addition, a sublayer of at least another type of collagen.

3. Biomaterial according to Claim 2, characterized in that the said sublayer comprises types I and III collagens.

4. Biomaterial according to any one of Claims 1 to 3, characterized in that the epidermal cells are keratinocytes.

5. Biomaterial according to any one of Claims 1 to 4, characterized in that the collagens are native or cross-linked collagens or mixtures of native collagen and cross-linked collagen.

6. Application of the biomaterial according to any one of Claims 1 to 5 in an in vitro evaluation test in toxicology, pharmacology or cosmetology.

7. Biomaterial according to any one of Claims 1 to 5, for the protection of wounds or burns or forming a healing agent.
